# EUROPEAN PATENT APPLICATION

(11) **EP 1 170 710 A1**
(43) Date of publication of application: **09.01.2002**
(21) Application number: 01500172.0
(22) Date of filing: 06.07.2001
(51) Int. Cl.: G08B 17/117, G01M 3/16

(54) **Safety device for combustible gases or liquids**

(30) Priority: 06.07.2000 ES 200001818
(71) Applicant: D'Urania Electrodomesticos Decoration Cocinas S.L., 46025 Valencia (ES)
(72) Inventor: Ortega Berruga, Jose Maria, 46025 Valencia (ES)
(74) Representative: Marques Alos, Fernando

(57) **Abstract**

**"SAFETY DEVICE FOR COMBUSTIBLE GASES OR LIQUIDS"**, of the type used in pipes for highly inflammable liquids and gases intended mainly for use in the home, shops, etc., made up of a detector or probe (1) which activates a sonorous alarm (4) upon detecting a leak, which has an electric plug (2) as well as a battery (3) which acts as an alternative energy source in case of an interruption in the electric power supply, and an electrovalve or other type of valve (5) connected directly to the detector or probe (1) which shuts off the liquid or gas pipe.

## Description

The objective of this invention, as expressed in the statement to this descriptive report, consists of a "**SAFETY DEVICE FOR COMBUSTIBLE GASES OR LIQUIDS**", of the type used for pipes carrying highly inflammable liquid or gaseous compounds, intended mainly for use in the home, shops, etc.

Today, there is growing social awareness with regard to achieving high safety standards in both the home and the workplace, with the aim of minimising the risk of accidents in these areas.

There are different types of detectors on the market which work as warning systems in the event of a leak.

To date however, the existing leak detectors on the market do not have an automatic shut-off feature to stop the flow of gas or liquid, therefore, in the event of a leak, serious accidents may result.

Similarly, if there is a power cut, these detectors usually fail to work with the risk of not detecting the gas or liquid leak in question.

As a result, a new device has been developed which solves the problems existing to date.

Basically, the new safety device for combustible gases or liquids is made up of a detector or probe which activates a sonorous or visual alarm upon detecting a leak.

It has both an electric plug and a battery which acts as an alternative energy source in case the electric power supply is interrupted.

Finally, it has an electrovalve or other type of valve connected directly to the detector or probe that shuts off the liquid or gas pipe.

The detector component in water pipes may consist of a buoy with contact, or with another mechanical system (paper or probe). In gas pipes, the detector will have a probe, paper or a heat sensor of fumes or gases; the components that fit together best in each case will be installed in the safety device.

This device is applicable to any household electrical appliance with an air extraction turbine, compressor or gas piping.

The new device operates in the following way:

When there is a leak, it is detected by the probe or gas detector, which activates simultaneously the sonorous or acoustic alarm and the pipe shut-off valve.

It may be very useful during holidays or when people are away as the device works automatically, closing off the supply pipe and activating an alarm in the event of a leak.

In addition, it can be installed on different devices with liquid or gas pipes.

### DESCRIPTION OF THE DIAGRAMS

To illustrate everything explained so far, a page of drawings is attached to this descriptive report, forming an integral part of it, representing the invention in a simplified and schematic way. These drawings are intended to be purely illustrative and are not an exhaustive representation of the practical possibilities of the invention.

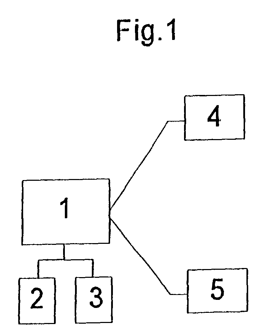

In these diagrams, figure 1 represents an outline of the new safety device for combustible gases and liquids.

### DESCRIPTION OF A PRACTICAL EXAMPLE

The new safety device for combustible gases or liquids is made up of a detector or probe (1) which activates a sonorous alarm (4) upon detecting a leak.

It has both an electric plug (2) and a battery (3) which acts as an alternative energy source in case the electric power supply is interrupted.

Finally, it has an electrovalve or other type of valve (5) connected directly to the detector or probe which shuts off the liquid or gas pipe.

Having established the concept behind the invention, the note of claims is drafted next, thus establishing the inventions that are the subject of the claims.

## Claims

1. **"SAFETY DEVICE FOR COMBUSTIBLE GASES OR LIQUIDS"**, of the type used in pipes carrying highly inflammable liquids or gases intended mainly for use in the home, shops, etc., characterised essentially because it is made up of a detector or probe (1) which activates a sonorous alarm (4) upon detecting a leak, which has an electric plug (2) as well as a battery (3) which acts as an alternative energy source in case of an interruption in the electric power supply, and an electrovalve or other type of valve (5) connected directly to the detector or probe (1) which shuts off the liquid or gas pipe.
